Europäisches Patentamt

European Patent Office

Office européen des brevets

(19)

(11) Publication number: **0 456 753 B1**

# EUROPEAN PATENT SPECIFICATION

(12)

(45) Date of publication of patent specification: **04.05.94**

(51) Int. Cl.⁵: **G01N 33/569**, G01N 33/58

(21) Application number: **90903488.6**

(22) Date of filing: **01.02.90**

(86) International application number:
**PCT/US90/00447**

(87) International publication number:
**WO 90/08959 (09.08.90 90/19)**

(54) **HIV p17 ASSAYS FOR MONITORING DEVELOPMENT OF AIDS.**

(30) Priority: **01.02.89 US 305318**

(43) Date of publication of application:
**21.11.91 Bulletin 91/47**

(45) Publication of the grant of the patent:
**04.05.94 Bulletin 94/18**

(84) Designated Contracting States:
**AT BE CH DE DK ES FR GB IT LI LU NL SE**

(56) References cited:
**EP-A- 0 246 829**
**EP-A- 0 293 792**
**WO-A-87/05399**

**INFECTION, vol. 19, 1991, suppl. 2, Appendix B; A. KLEINSCHMIDT et al., pp. 589-592&NUM;**

**AIDS, vol. 1, no. 3, 1987, Gower Academic Journals Ltd., J.M.A. LANGE et al., pp. 155-159, abstract&NUM;**

**THE LANCET, vol. II, 26 July 1986; J. GOUDS-MIT et al., pp. 177-180&NUM;**

**ANNALS OF INTERNAL MEDICINE, vol. 108, 1988, American College of Physicians; G.G. JACKSON et al., pp. 175-180&NUM;**

(73) Proprietor: **E.I. DU PONT DE NEMOURS AND COMPANY**
**1007 Market Street**
**Wilmington Delaware 19898(US)**

(72) Inventor: **DEVASH, Yair**
**31 Park Hill Ter.**
**Princeton Junction, NJ 08550-1944(US)**
Inventor: **REAGAN, Kevin, James**
**3 Dehoff Dr.**
**Flemington, NJ 08822-1939(US)**

(74) Representative: **von Kreisler, Alek, Dipl.-Chem. et al**
**Patentanwälte**
**von Kreisler-Selting-Werner**
**Postfach 10 22 41**
**D-50462 Köln (DE)**

**Description**

This invention relates to detection of human immunodefiency virus (HIV) infection, and more specifically to a method of immunoassay employing a core antigen of HIV as a prognostic means for monitoring the development of aquired immune deficiency syndrome (AIDS).

Background

HIV has been firmly established as the etiologic agent of AIDS. The genomic structure of HIV resembles that of all of the replicating retroviruses, consisting of three essential genes. These genes are: the gag gene, coding for the major nonglycosylated viral core proteins, p15, p17, p24 and p55; the pol gene, coding for reverse transcriptase; and the env gene, coding for the glycoproteins of the viral envelope, gp 160, gp120 and gp41. In addition, at least four functional nonstructural genes have been identified. The gag gene is expressed as a precursor protein (p55) that is proteolytically processed to p17, p24 and p15. The expression of the gag gene as a p55 precursor indicates that each of the core proteins is produced in equimolar concentration. The recombinant synthesis of said p55 precursor protein is disclosed in the EP-A-0 293 792. Similarly, the env gene is expressed as a precursor glycoprotein (gp160) which is proteolytically processed to produce equimolar amounts of gp41 and gp120.

Presence of antibodies against HIV proteins (antigens) in the sera of most AIDS and AIDS related complex (ARC) patients, and in sera of asymptomatic individuals infected with the virus, has made possible the development of immunologically based tests that detect the antibodies and indicate HIV infection. These tests have been used to block the spread of HIV through transfusion by identifying blood samples from donors infected with the virus, and also for diagnosis of HIV infection.

Commercially available HIV specific antibody tests for the purposes of screening and diagnosis were initially based on the use of proteins from the lysates of inactivated virus as antigens. However, the availability of purified, synthetic and recombinant viral proteins and protein fragments has offered new approaches to the development of more specific and sensitive tests for diagnosis and prognosis of HIV infection.

Fluctuations in the level of specific HIV antibodies in infected individuals have been shown to correlate with the progression of the disease from asymptomatic to symptomatic stages. Studies showed that serodiagnostic profiles in serum as well as cerebrospinal fluid were strongly associated with the present clinical status of the patient. Furthermore, the studies suggested that such profiles might be useful in predicting the clinical outcome of asymptomatic individuals infected with HIV. For example, the levels of antibody to p24 and antibody to gp41 in patients at the asymptomatic stage were consistently elevated. With the progression of the disease to AIDS, the level of antibody to p24 declined or disappeared [Schubach et al, N Eng. J. Med Vol. 312, 265-270, 1985; Goudsmit et al, J. Virol. Methods, Vol. 17,19-34, 1987, and; Allain et al, Br. Med. J. Vol. 229, 228-230, 1986].

The decline of the antibody reactivity to the outer viral core protein p17 has also been detected by the Western immunoblot assay, and the potential clinical importance of antibody to p17 has been suggested. [Lange et al, AIDS Vol. 1,155-159, 1987; Schulte et al., Klin Wochenschr 66,(11), 1988]. However, antibody to p17 is not generally regarded as a reliable indicator of HIV infection, due to a large number of false positive p17 results in Western blot test. Moreover, since Western blot is a semi-quantitative test at best, it is not expected to provide an early indication of a drop in antibodies.

Assays have been developed which detect and measure p24 antigen of HIV. These assays are heterogeneous sandwich ELISA methods, in which capture antibody immobilized on a solid surface binds HIV p24 from a sample of a biological fluid, detection antibody which is labeled directly or indirectly with an enzyme binds to the captured antigen to form an antibody-antigen-antibody sandwich, the enzyme in the sandwich acts on a substrate solution to produce color in the solution, and the absorbance of the solution is measured and correlated to the presence or quantity of antigen in the sample.

One such assay, marketed by DuPont, utilizes a polyclonal rabbit anti-p24 antibody for both capture and detection. Another, marketed by Abbott, utilizes polyclonal human anti-HIV antibody for capture and a polyclonal rabbit anti-HIV antibody for detection. The antibody used in the DuPont assay for both capture and detection is produced by immunizing a rabbit with purified p24 and selected for reactivity to purified p24, so the assay is highly specific for p24. Both the DuPont and the Abbott assay are highly sensitive.

Sensitive immunoassays for detection of the p24 antigen allowed invesigators to establish a strong correlation between serum/plasma levels of p24 and clinical outcome of patients [Goudsmit et al, Lancet Vol. II, 177-180, 1986]. Decreasing levels of antibody to p24 as determined by the antibody assays and corresponding increasing level of p24 antigen as detected by the antigen assays strongly indicate progression toward full-blown AIDS. Thus, the p24 antigen assay as well as the antibody assays have become important tools for determining the stage of disease due to HIV infection. For example, the HIV p24 antigen levels in the serum of AIDS patients treated with Azidothymidine (AZT) declined with remission, indicating the importance of the antigen assays as tools for monitoring the antiviral drug therapy [Chaisson et al, Lancet, Correspondence Vol. 315, No. 25]. The HIV antigenemia was primarily associated with the p24 viral core protein [Jackson et al, Annals of Internal Medicine Vol.108, 175-180, 1988; Forster et al, AIDS Vol.1,235-240, 1987].

Presence of antibody to p24 in a biological fluid inhibits the p24 antigen assay, because the endogenous antibody competes with the capture antibody of the assay for binding to the endogenous p24 antigen. The competition between endogenous antibody and capture antibody has made it possible to use the p24 antigen ELISA in combination with exogenous p24 in a competitive assay to determine the level of anti p24 in human plasma/serum. For example, in one reported experiment (Huskins and Malone, Poster Presentation, Haemonetics Research Institute, 12th International Apheresis Symposium, April 27-29, 1987), investigators determined the absorbance produced in the p24 ELISA by a sample of p24 in buffer at 1 ng/mL. Serial dilutions were made of several plasma/serum samples, and p24 was added to a concentration of 1 ng/mL, then the absorbance of each dilution was determined in the p24 ELISA. A curve of dilution vs. absorbance was obtained for each plasma/serum sample. The dilution which produced an absorbance equal to 50% of the absorbance produced by the 1 ng/mL p24 solution was defined as the 50% inhibition titer of the sample. For ten plasma/serum samples tested, 50% inhibition titers ranged from 600 to 3200. In another reported experiment (Croxson et al., IV International Conference on AIDS, Book 2, Abstract Nol 1638 (June, 1988) the competition assay using exogenous p24 and the p24 antigen ELISA was used to follow the serum anti-p24 antibody level in 38 HIV seropositive patients. The antibody level was expressed as p24 binding capacity, i.e. the capacity of the serum to bind exogenous (added) p24 as determined by the p24 ELISA. The same p24 ELISA was used to follow the serum p24 levels in the same patients.

Prior to the identification of the HIV virus, surrogate markers were utilized in an attempt to identify blood exposed to HIV. For example, early studies of AIDS patients indicated that the level of alpha-1-thymosin, a thymic hormone, was elevated in blood samples of AIDS pateints as measured by radioimmunoassay (RIA). When the sequence data for HIV became available, it was apparent that there was a sequence homology between the hormone and the C-terminus of the outer viral core protein, p17 [McClure et al, J. Immunol Vol. 128, 368-373, 1982; Naylor et al, Proc. Natl. Acad. Sci. US Vol. 84, 2951-2955, 1987]. Naylor et al. suggest that the 50% homology between alpha-1-thymosin and p17 would result in identification of p17 as the viral protein that cross-reacts in the alpha-1-thymosin RIA. Naylor et al. immunized rabbits with a 30 amino acid synthetic peptide analog of p17 and with alpha-1-thymosin and found that the anti-sera were not cross-reactive. Naylor et al. suggest that this lack of cross-reactivity could form the basis of an assay to measure the HIV protein in the presence of serum alpha-1-thymosin, and that quantitive information about the p17 levels in serum may be useful to monitor HIV infection in seronegative individuals.

European Patent Application Publication No. 0 246 829 discloses a heterogeneous sandwich ELISA for detecting p17 in a patient's serum. A microtiter plate is coated with antibodies to alpha-1-thymosin or to a peptide displaying the antigenicity at position 91 to 109 of p17. A sample of serum is added and after incubation, the plate is washed. A preparation of the same antibodies, linked to an enzyme is added, and after incubation, the plate is again washed. Enzyme substrate solution is added and after a further incubation, the absorbance is measured.

Prior to this invention, it was not predictable whether p17 would be a valuable prognostic marker for the clinical management of HIV seropositive patients. Since p24 and p17 are produced in equimolar amounts by proteolytic cleavage of p55, it was possible that the levels of the two proteins in a patient's body fluid would be substantially the same at all times, so that monitoring the level of p17 would not provide any more information of prognostic value than monitoring of p24 alone. On the other hand, it was not known whether these proteins are equally stable. While p24 is phosphorylated, a very unusual feature for a major retroviral core protein, p17 is myristylated as are all NH2-terminal gag proteins of the known human retroviruses. [Ratner et al, Nature Vol. 313, 277-284, 1985]. P24 is always a much stronger band than p17 in the SDS-polyacrylamide gel electrophoresis pattern of purified viral preparations, which suggests that p24 survives the purification process better than p17. In addition, it was not known whether p17 would be cleared from the body fluids more or less rapidly than p24, or whether the relationship would be the same in all infected individuals. It was also not known to what extent the p17 antigen might be masked by complexing with endogenous anti-p17 antibody and thus unavailable for providing information of prognostic significance. It was also not known what effect antiviral drug therapy would have on p17 antigenemia. Such factors, along with the concern for p17 crossreactivity with alpha-1-thymosin, may account for the fact that p17 has not been recognized as a useful prognostic marker for monitoring development of AIDS.

## Summary of the Invention

This invention is based on the discovery that, although p17 and p24 are produced in equimolar amounts, they do not track each other in all patients. The antigenemia and binding capacity measurements disclosed herein indicated an independent pattern in approximately 30% of AIDS patients. This may be due to different immunogenicity, stability, and reactivity of the proteins detectable in some infected individuals.

In one aspect, this invention is a method of monitoring and prognosticating the disease state of an individual who is seropositive for antibodies to HIV which comprises intermittently determining the HIV p17 antigen level in the individual's blood serum or other body fluid. In another aspect, this invention is a method of monitoring and prognosticating the disease state of an individual who is seropositive for antibodies to HIV which comprises intermittently determining the level of free complexing antibody to p17 in the individual's blood or other body fluid. In a preferred aspect of the invention, the individual's body fluid is simultaneously monitored for both level of p17 and free complexing antibody to p17, i.e. antibody which is capable of complexing with and masking exogenous (added) p17 in the binding capacity assay described below.

We have found that in some patients, p17 antigen and free complexing anti-p17 antibody level measurements indicate a poor prognosis when p24 antigen and free complexing anti-p24 antibody level measurements do not, and vice versa, and that a poor prognosis derived from either p17 antigen/antibody measurement or p24 antigen/antibody measurement usually correlates to an adverse outcome. Therefore, to obtain information of maximum prognostic significance, it is desirable to simultaneously monitor both p24 and p17 antigen levels (separately or as the sum of the two antigens) and both free complexing anti-p24 antibody and free complexing anti-p17 antibody levels.

Although various assay formats could be used to monitor level of p17 antigen and free complexing anti-p17 antibody, it is preferred to use a heterogeneous sandwich ELISA for measuring the antigen and to use the same ELISA in conjunction with exogenous p17 in a binding capacity assay for monitoring free complexing anti-p17 antibody. The ELISA can be specific for p17 antigen or it can be a combination p17/p24 ELISA. The p17 specific ELISA utilizes antibody for capture and/or detection which binds only p17 antigen. The p17/p24 ELISA additionally utilizes antibody for capture and/or detection which binds only p24. When a p17/p24 ELISA is used, an analogous p24 specific ELISA is used simultaneously on a separate sample, and the difference in absorbances in the two assays is a measure of the p17 level in the test fluid.

In the binding capacity assay to determine the level of free complexing antibody to p17, a known concentration of exogenous p17 is added into a sample of the patient's serum or other body fluid, the mixture is incubated to permit the added antigen to complex with any free complexing anti-p17 antibody in the fluid, then the quantity of any remaining free p17 antigen is determined by means of the p17 or p17/p24 ELISA. The difference between the amount of exogenous p17 added and the amount detected after incubation (recovered) is the amount bound and is directly proportional to the level of free complexing anti-p17 antibody in the fluid. If the exogenous antigen is purified native, synthetic or recombinant p17, the combination p17/p24 ELISA can be used, and the binding capacity assay will be specific for anti-p17 antibody, due to specificity of the antigen.

The p24 binding capacity of the test fluid can be determined simultaneously using the known p24 binding capacity assay in which the added antigen is HIV lysate, or using a modification thereof in which the added antigen is purified native, synthetic or recombinant p24.

## Brief Description of the Figures

Fig. 1 Western blot analysis of capture and detector antibodies used in p24, p17 and p24/p17 Antigen ELISA assays. The description for each lane is described as follows:

Lane 13:  Negative Control
Lane 9:  p24 Rabbit Antibody Conjugate
Lane 5:  Rabbit Anti-HIV Conjugate (#483)
Lane 11:  Rabbit #483 + p24 Antibody Conjugate
Lane 4:  p17 Rabbit Antibody Conjugate (#892)
Lane 18:  Human Anti-HIV Capture Antibody (KRH-4).

Fig. 2 Standard curves for p24 and p17 by p24 (Fig. 2a), p17 (Fig. 2b), and p24/p17 (Fig. 2c) Core Antigen ELISA Assays.

Figs. 3-5 Analyses of Core Antigens and Core Binding Capacities from sequential bleeds of HIV infected individuals #212 (Fig. 3), #18 (Fig. 4) and #8 (Fig. 5).

4

Detailed Description

The HIV p17 antigen ELISA used in this invention is a typical heterogeneous sandwich immunoassay consisting of a capture antibody bound to a solid phase and a detector antibody labeled with an indicator enzyme. The capture antibody may be human anti-HIV, or poly- or mono-clonal antibody generated by immunizing an animal with viral lysate or purified native, synthetic or recombinant HIV p17 gag protein or a fragment thereof. The solid carrier may be a water insoluble organic substance such as a polymer or copolymer, an inorganic substance such as silica, zeolite, and glass, or any other water insoluble material suitable for use in ELISA. The solid carrier may be porous or non-porous, and may be made in any shape or form. The antigen from a sample suspected of containing HIV p17 is then captured onto the solid support. The sample may be a serum, plasma, cerebospinal fluid, in vitro culture fluids or any other body fluid. The captured p17 is detected by addition of a labeled second antibody also specific for HIV p17. The detector antibody may also be a poly- or mono-clonal antibody, and may be directly labeled with an enzyme such as horseradish peroxidase, alkaline phosphatase, or indirectly labeled as in double receptor assays. For example, the second antibody may be biotinylated and detection made by the addition of an avidin-enzyme conjugate. A double receptor assay can also be formatted using a labeled species specific third antibody recognizing the second antibody of the ternary sandwich complex. The enzyme may be any one of commonly used labels in ELISA or other enzymes having properties desirable as a label. Measurement of the enzyme can be made either chromogenically or fluorogenically using appropriate substrate and buffer systems for the enzyme. The signal generated in this immunoassay is directly proportional to the amount of p17 antigen captured. The sandwich assay may be run in the forward, reverse or simultaneous format; the forward format is preferred. The p17 antigen ELISA assay may be used alone or in combination with other ELISA for HIV antigens such as p24.

The HIV p17 binding capacity assay is a measurement of a subpopulation of p17 antibodies which are capable of complexing with p17 in solution and masking it from detection by the p17 antigen assay. Traditionally, the antibody levels are detected by measuring the captured antibody on a large excess solid phase antigen. This is an indication of total antibody of weak to high affinity. The binding capacity assay described herein measures the antibody utilizing the p17 antigen assay as a means for detection. Therefore, the binding capacity assay is designed more specifically to access the level of the higher affinity antibody population for p17.

The binding capacity assay used in this invention comprises the steps of:
1) diluting a sample suspected of containing the antibody for HIV p17 to provide a convenient volume, ;
2) contacting the diluted sample with a solution containing a defined quantity of HIV p17 antigen to form a reaction mixture;
3) incubating the mixture to allow p17 to complex with the anti-p17 antibody in the sample;
4) subjecting the mixture of step (3) as a sample to perform an immunoassay by the method of ELISA to determine the level of non- complexed p17 antigen in the mixture; and
5) estimating the binding capacity of the sample for p17 by comparison of the p17 antigen level determined in step (4) with the seronegative and/or seropositive control sample values.

The sample may be a serum, plama, cerebrospinal fluid, tissue extract, animal serum or any other living body fluid. The sample may be diluted with a commonly used kit sample diluent or with a protein-based buffer. The HIV p17 antigen may be a recombinant protein, purified native protein or their fragments, or synthetic peptides containing immunodominant epitopes of p17. The antigen ELISA method is that described above for HIV p17. When the p17 antigen other than the native antigen is to be used to form immune complexes with the patient antibody in the sample, the antigen is to be screened to ensure reactivity with human anti-p17 antibody as well as with p17 antibodies used in the antigen ELISA.

Various assay formats designed to measure the level of free complexing antibody may also be used, for example, radioimmune precipitation assay (RIPA), competitive ELISA or any other methods known to one skilled in the art.

The ELISA and binding capacity assay for HIV 17 antigen and antibodies of this invention may be used in conjuntion with the corresponding assays for HIV p24 for more reliable detection of HIV infection from asymptomatic to symptomatic patients. The binding capacity assay for p17 is more specific to a higher affinity subpopulation of the p17 antibdy than is the Western blot and is thus capable of detecting the degradation of the patient's immune status at an earler time.

The experimental work which led to the making of this invention will now be described.

Materials and Methods:

Patient populations: Samples subjected to analysis were obtained from a variety of sources. The majority of defined HIV-symptomatic cases were received from Graduate Hospital in Philadelphia. Seronegative samples were obtained from either employee donors or the Blood Bank of Delaware. All samples were handled under BSL2 safety conditions as outlined by DuPont Glasgow, Delaware Site policy. Initial serological evaluation was performed by the DuPont HIV Antibody ELISA with confirmation by western blotting of serum using the DuPont/Biotech kit as described below.

Western Blot Procedure: HIV viral proteins or recombinant p17 were electrophoresed on a 12% SDS-polyacrylamide gel and transferred electrophoretically to nitrocellulose sheets using a transfer buffer of 0.012M Tris (pH 8.3) with 0.096M glycine and 20% methanol. Electrotransfer was conducted for 1 hour at 30 volts. The nitrocellulose sheet was blocked for at least 1 hour with a blotto solution (5% nonfat dry milk dissolved in PBS) (Johnson et al., Gene Anal. Techn., 1:3, 1984). Blotto solution was also used as the carrier for the primary and secondary antibodies. The nitrocellulose sheet or strips cut from that sheet was incubated with the primary antibody for at least 2 hours at room temperature, washed 4 times with PBS containing 0.05% tween-20, then reacted with an HRP-conjugated anti-species antibody for 1 hour, and washed again in PBS/T. Bound antibody was detected upon the addition of the substrate, 4-chloro-1-napthol. After the color developed on the strips, the reaction was stopped by rinsing the strips with deionized water, the strips dried and stored in the dark.

HIV p24 Core Antigen Detection: HIV p24 antigen was detected by the antigen capture enzyme immunoassay using the instructions provided in the DuPont p24 ELISA system. The overnight sample capture version was preferentially used with quantitative values determined using a standard curve of whole viral lysate whose p24 protein concentration was calibrated against affinity purified p24 antigen as described by the manufacturer. The Abbott HIV Antigen ELISA was performed as described by the manufacturer.

HIV p24/p17 Core Antigen Detection: A combined p24/p17 antigen capture assay was devised. 96-well Nunc microwells were coated with human anti-HIV at a concentration of 10 ug/ml in carbonate/bicarbonate buffer, pH 9.6. After an overnight coating at 4 degrees C, the plates were blocked with 2% casein prepared in PBS, pH 7.5, and plates stored in this blocking buffer in the cold until used. In this assay the soluble core antigens in standards, tissue culture fluids, serum, etc. containing 0.5% Triton X-100 were captured during an overnight incubation at room temperature. The detection antibody was rabbit anti-HIV raised by hyper-immunization of New Zealand White rabbits with manufacturing grade HIV-1 lysate (DuPont) and screened for p24 and p17 specificites by Western blot. The antibody was purified and biotinylated using commercially available N-hydroxysuccinimide biotin. The biotinylated antibody was then diluted with a HIV p24 kit conjugate (rabbit polyclonal anti-p24 antibody conjugated to biotin) to maximize p24 sensitivity. Incubation was allowed to proceed for 2 hours at 37 degrees C. An HRP conjugated streptavidin was then added to washed wells for 1/2 hour at 37 degrees C to complex with the bound detector antibody. Following additional washes, a colored product was formed during incubation with O-Phenylenediamine substrate solution. A standard curve generated by either viral lysate or recombinant p17 (described below) was prepared in standard sample diluent. The amount of color generated was directly proportional to the amount of antigen captured.

HIV p17 Core Antigen ELISA: A sandwich immunoassay similar in design to the p24 antigen ELISA described above, allowed specific detection and quantitation of HIV p17. The capture antibody bound to the solid support was an HIV p17 specific monoclonal antibody, BT2, available commercially (New England Nuclear Research Products). The antigen, captured from the sample was detected by the addition of a p17 specific rabbit polyclonal antibody. The detection antibody was raised by immunizing rabbits with purified recombinant p17 and was purified and biotinylated. Detection was made by the addition of Avidin-HRP conjugate as described for p24 ELISA.

HIV p17 recombinant (rec.) protein: Plasmid pGAG17, which specifies the expression in E. coli of the mature, unfused p17 protein, was consturcted by extending the gag coding sequence from the Pvu II site at nucleotide 692 of Ratner, et al., Nature 316, 277, 1985, with a synthetic oligonucleotide linker which introduces a stop codon after the TYR codon at the p17-p24 junction of the p55 precursor protein. In pGAG17, the coding sequence corresponding to nucleotides 334 to 729 of the BH10 isolate of HIV-1 is under transcriptional and translational control of the lambda P1 promoter. E. coli induced with a temperature shift expresses p17 in soluble form at at levels of 5% of total soluble protein. Purification of p17 was achieved by ammonium sulfate fractionation , size exclusion chromatography, cation exchange chromatography and reverse phase HPLC. The purified protein had a pI of 6.5 to 7.0 and yielded the expected sequence by Edman degradation and carboxypeptidase Y digestion. A description of plasmid pGAG17 is included in commonly-assigned patent application 07/057569, filed June 1,1987, the disclosure of which is

EP 0 456 753 B1

incorporated herein.

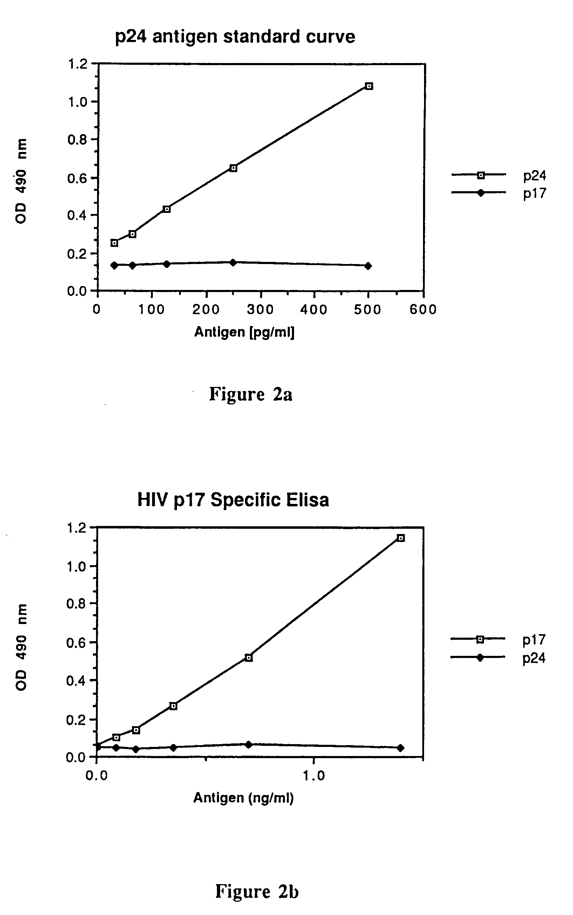

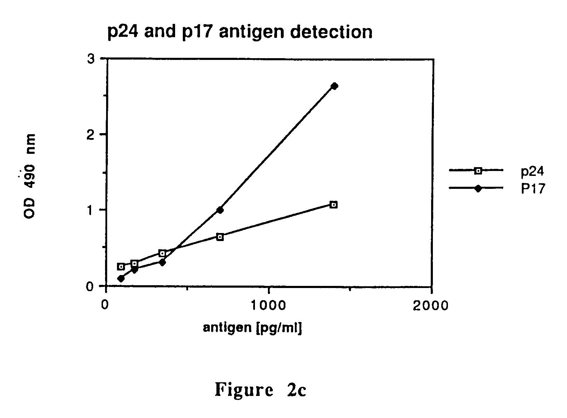

Binding Capacity (BC) Assays: Quantitative measurement of the capacity of serum samples to complex and mask the detection of either HIV p24 or p17 antigen was determined in separate assays. Serum samples were diluted 1:50 for p24 and 1:20 for p17 in the DuPont HIV p24 Core Antigen ELISA Kit sample diluent prior to the addition of either p24 at 0.5 ng/ml or rec. p17 (diluted to 1:400K). The final serum dilution (after combination with antigen) was 1:250 for p24 BC and 1:100 for p17 BC. Samples were held at 37 degrees C for 30 min prior to addition of 200ul to the wells of the antigen plates (either the HIV p24 ELISA kit for a p24 BC assay or the HIV p24/p17 ELISA kit for a p17 BC assay). Thereafter, the concentration of recovered antigen (i.e. unbound antigen in solution after incubation with sample) was measured using the HIV p24 ELISA or the HIV p17/p24 ELISA as described above. Standard curves (Figure 2) were established for each assay. Controls consisted of aliquots of sera with known p24 or p17 BC and known seronegative samples. The quantitative p24 or p17 BC (Relative Binding Capacity) was calculated as: [Ag1] - [Ag2] x dilution factor, where Ag1 is the initial concentration of p24 (ng/ml, unless stated otherwise) or p17 (O.D. units/ml, unless stated otherwise), Ag2 is the recovered concentration of each antigen in similar unit measurement.

Results:

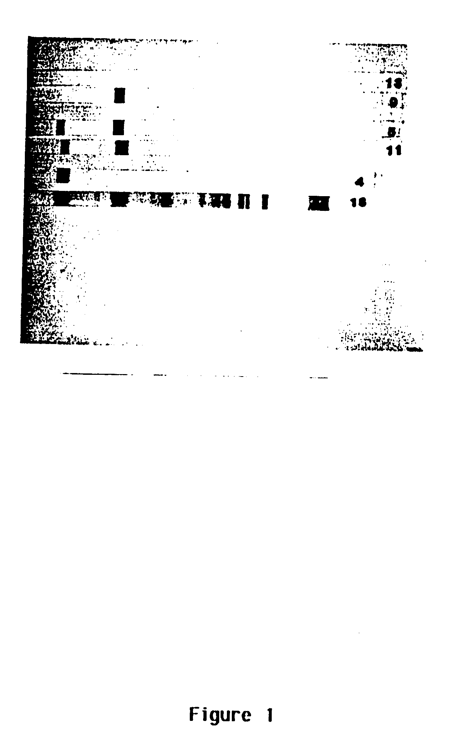

Characterization of the antibodies used to capture and detect p17 and p24: The reactivity of rabbit polyclonal antibody (#483, 892) to HIV proteins and the human anti-HIV serum (KRH4) was established for p24 and p17 by Western blot as shown in Figure 1.

The rabbit and human antibodies discussed above were formatted into the p24, p17 and 17/p24 antigen assays because of their ability to detect and capture either or both p24 and p17.

HIV p24, p17 and p17/p24 Antigen ELISA Standard Curves: In order to document the specificity of each antigen ELISA (p24 and p17), standard curves comprised of known purified antigen (p24 or recombinant p17) were generated. Figures 2a and 2b demonstrate specificities to p24 and p17 only, respectively, while Figure 2c demonstrates detection of both antigens.

Abbott HIV Antigen ELISA: In order to determine whether a commercially available test is capable of reacting with p17 containing samples, the HIV Antigen ELISA, Lot # 22464M200 (Abbott, North Chicago, IL) was compared to the p24/p17 Antigen ELISA for the detection of recombinant p17 and human sera shown to have low or absent p17 Binding Capacities. We tested the Abbott assay since its manufacturers have claimed that it is primarily sensitive for p24 yet reactive with other viral antigens (Paul, D., Falk, L. J. Cell Biochem. Suppl. 10A:224, 1986). We have also compared reactivity to the DuPont HIV p24 Core Antigen ELISA which claims only p24 reactivity. The data (TABLE 1) clearly indicate that although the Abbott assay performed within its specifications for its positive and negative controls, reactivity was not seen with the p17 recombinant protein nor with any human serum except that which contained HIV p24 antigen (as defined by the specificity of the DuPont p24 ELISA) (patient # 18). The HIV p24/p17 Core Antigen ELISA reacted well with the p17 recombinant and showed signal with all but one of the human serum.

7

TABLE 1

| COMPARISON OF THE HIV p24/p17 CORE ANTIGEN ELISA TO THE ABBOTT HIV CORE ANTIGEN TEST AND THE DUPONT HIV p24 CORE ANTIGEN TEST | | | | |
|---|---|---|---|---|
| SAMPLE | AMOUNT | OPTICAL DENSITY | | |
| | | ABBOTT ELISA* | DUPONT HIV p24** | HIV p24/p17*** |
| POS. CNTRL<br>NEG. CNTRL | | 0.569<br>0.035 | 1.58<br>0.028 | 2.78<br>0.033 |
| rec. p17 | 2 ng/ml<br>1<br>0.5<br>0.25<br>0.125 | 0.041<br>0.035<br>0.024<br>0.03<br>0.03 | 0.033<br>0.039<br>0.037<br>0.022<br>0.036 | 3.677<br>2.93<br>1.151<br>0.378<br>0.208 |
| HUMAN SERA (DISEASE) | | | | |
| KRH4 (ASYMPT.) | | 0.024 | 0.039 | 0.048 |
| 194 (AIDS) | | 0.048 | 0.1 | 0.242 |
| 194F (AIDS) | | 0.06 | 0.095 | 0.220 |
| 269 (AIDS) | | 0.03 | 0.073 | 0.526 |
| 24A (AIDS) | | 0.013 | 0.066 | 0.597 |
| 18P (AIDS) | | 0.11 POS. | 0.192 | 1.222 |
| 7007029 (AIDS) | | 0.05 | 0.095 | 0.657 |

\* CUTOFF = 0.085
\*\* CUTOFF = 0.128
\*\*\* CUTOFF = 0.133

In order to confirm that the Abbott assay had poor if any p17 reactivity, the rabbit anti-HIV detector antibody used in the Abbott test kit was used in a standard western blot using as the antigen, recombinant p17. The data (not shown) documented that in comparison to the detector antibody in the p24/p17 Antigen ELISA, the Abbott detector failed to react with recombinant p17 and thus explained the lack of reactivity to p17 shown in the table above. As expected, the detector in the DuPont p24 Antigen ELISA failed to react with p17.

Independence of HIV p24 and p17 Binding Capacities. In order to investigate whether a p17 Binding Capacity Assay could be constructed and yield clinically useful information, an assay was formatted. The existing HIV p24 Binding Capacity Assay uses HIV viral lysate, containing all the HIV proteins found in that partially purified virus, which are added to a diluent of sample antibody. The mixture of antigen and antibody is then transferred to the p24 Antigen ELISA and uncomplexed p24 antigen from the added lysate is quantitated. For the preliminary analysis, we have used the p24 Binding Capacity assay already optimized on the HIV p24 Core Antigen ELISA. The p17 Binding Capacity was performed using a non-purified p17 recombinant on the p24/p17 Core antigen ELISA. Data is represented in units of optical density. Shown below is the result of human sera grouped by disease status for binding capacity.

TABLE 2

| P24 AND P17/P24 BINDING CAPACITIES OF HIV-INFECTED INDIVIDUALS. | | | |
|---|---|---|---|
| SAMPLE | DIAGNOSIS | p24 BC (units/ml)* | p17 BC (units/ml)** |
| B524 | seroneg. | 0 | 51.1 |
| B525 | " | 0 | 6.1 |
| B544 | " | 0 | 11.6 |
| B548 | " | 0 | 5.9 |
| B546 | " | 0 | 8.3 |
| B543 | " | 0 | 11.4 |
| B547 | " | 0 | 4.2 |
| B552 | " | 0 | 0 |
| B526 | " | 0 | 7.4 |
| B549 | " | 0 | 13.5 |
| 113 | asymptom. | 305.5 | 55.4 |
| 203 | " | 329.5 | 61.2 |
| 260 | " | 320.75 | 98.7 |
| 99 | " | 150.2 | 35.5 |
| 296 | " | 19 | 2.6 |
| 121 | " | 280.2 | 39.9 |
| 243 | " | 243.75 | 46.2 |
| 289 | " | 326.25 | 26 |
| 302 | " | 301.75 | 88.5 |
| 130 | " | 153.5 | 13.5 |
| 136D | AIDS | 15.5 | 3 |
| 181B | " | 43.25 | 0 |
| 30M | " | 8.5 | 99 |
| 136F | " | 0 | 0 |
| 24A | " | 222.25 | 0 |
| 183B | " | 0 | 0 |
| 59D | " | 12.25 | 0 |
| 30C | " | 0 | 98.2 |
| 6D | " | 0 | 0 |
| 183H | " | 214.25 | 0 |

\* MAX p24 BC = 349
\*\* MAX p17 BC = 100.8

The p24 and p17 BC showed the same trends with seronegative samples demonstrating no or little ability to mask antigen from detection. Healthy seropositive individuals had a stronger ability to mask antigen for either p24 or p17, an ability which on average was lost among those classified as having clinical AIDS. A number of samples tested resulted in p24 and p17 BC discrepency. In particular, samples 289, 24A, and 183H show potent p24 BC in the absence of p17 BC. Other samples such as 30M and 30C show the opposite phenomena where a strong p17 BC is apparent in the absence of p24 BC. These data demonstrate that the two antibody populations as measured by the BC method have independent patterns.

We have concluded that in spite of the fact that the two gag gene products come from the same precursor molecule and thus must be produced in equimolar amounts, the occurrence of antigen and complexing antibody vary independently of each other.

TABLE 3 shows that half (8/16) of the patients which demonstrated strong anti-p24 antibodies and no anti-p17 antibodies in Western blots, scored positive for p17 antigen. All 16 patients were negative for p24 antigen. The results indicate that p17 antibody depletion is due at least in part to the increase in p17 antigen masking the antibody detection. In this population of seropositive patients, monitoring by p24 antigen and BC only would have missed those that exhibited p17 antigenemia.

**EP 0 456 753 B1**

## TABLE 3:  ANTIGENEMIA AMONG SAMPLES SHOWING WEAK OR ABSENT P17 AND STRONG P24 BANDS BY WESTERN BLOTS

p24 antigen Elisa

|  | + | | - |
|---|---|---|---|
| p24/p17 antigen Elisa | | | |
| + | 0 | | 8 |
| - | 0 | | 8 |

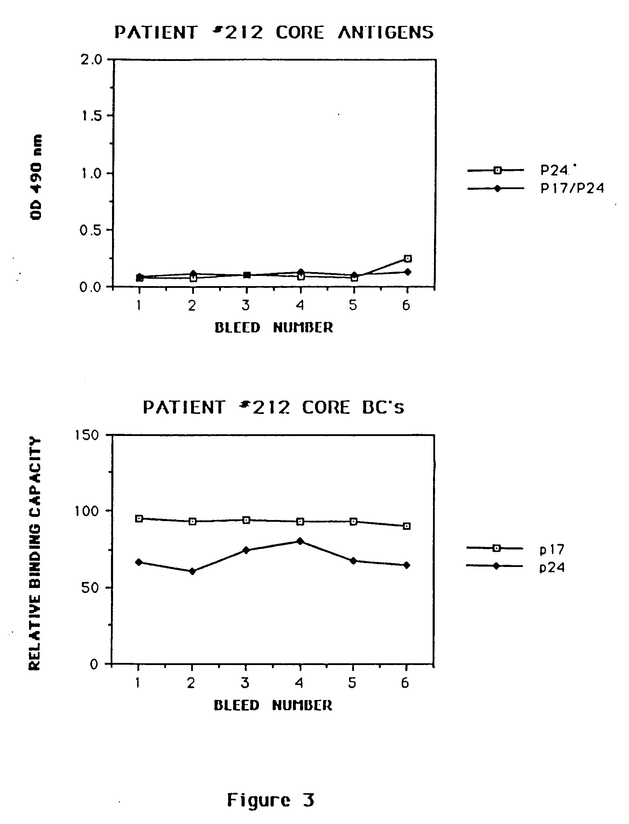

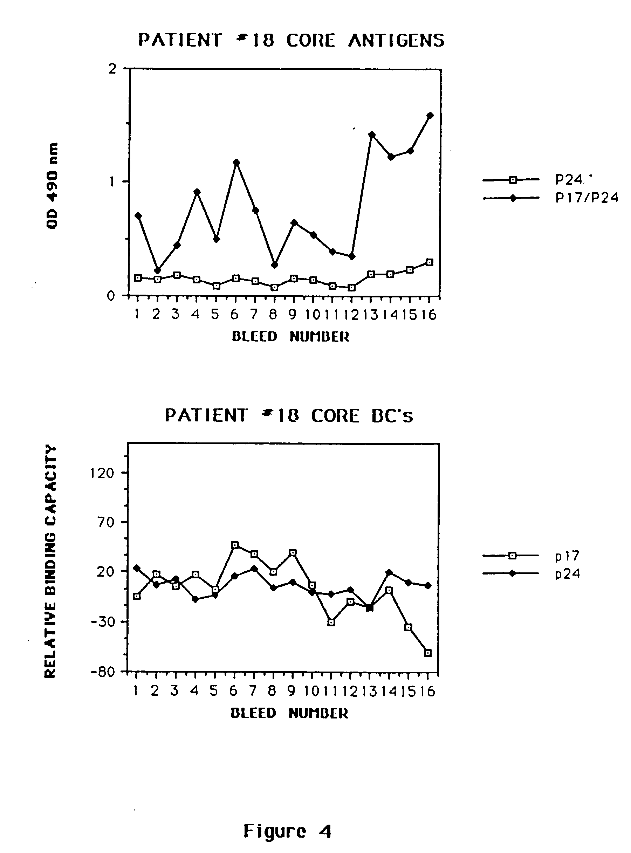

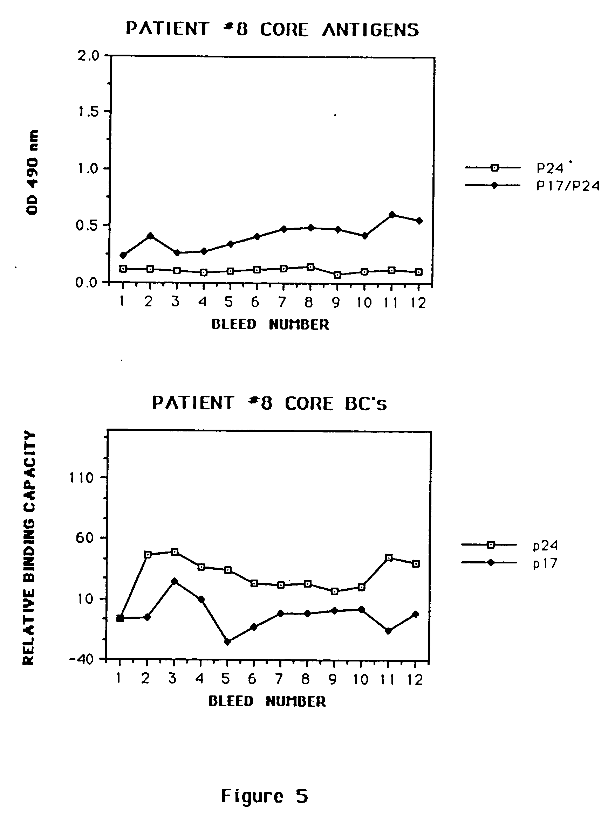

HIV p24 and p17 Antigen and Binding Capacity Values from Selected Patient Samples: p24 and p17 antigen and binding capacity were measured as described in materials and methods in 13 patients. The data shown in Figures 3-5 are presented as optical densities (OD) of antigen ELISA's and relative binding capacity values. Although only 3 patient results are shown here to represent the types of patterns noted, 8 out of 13 patients showed similar patterns for p24 and p17. Thus 38% of the cases (5/13) showed an independent pattern.

TABLE 4 summarizes examples of the various combinations of p24 and p17 antigen expression and binding capacities in selected patient samples, including those shown in Figures 3-5.. An absence in either or both binding capacities or presence of the p17 antigen level is associated with poor prognosis. Data for patients who exhibited p24 antigenemia are not shown, since p24 is well established in the art as the prognostic indicator.

TABLE 4

| CORRELATION OF PATIENT P17 AND P24 ANTIGEN AND BINDING CAPACITY VALUES WITH PROGNOSIS AND AZT RESPONSE | | | | | |
|---|---|---|---|---|---|
| Patient # | Diagnosis | P24 Antigen | P17 Antigen | P24 BC | P17 BC |
| 212 | AIDS, Good AZT response | - | - | + + + | + + + |
| 18 | AIDS, KS, Poor AZT response, Expired | - | + | + /- | + /- |
| 8 | AIDS, CMV, Poor AZT response Expired | - | + | + | - |
| 183* | AIDS, Poor AZT response, Expired | - | - | + + | - |

* Patient #183 represents a single bleed. Other patient results correspond to Figures 3-5.

**Claims**

1.  A method of monitoring and assessing the development of AIDS in an individual who is seropositive for antibody to HIV which comprises intermittently determining the HIV p17 level in a body fluid of the individual.

**2.** A method of monitoring and assessing the development of AIDS in an individual who is seropositive for antibody to HIV which comprises intermittently determining the level of free complexing anti-p17 antibody in a body fluid of the individual.

**3.** Method of claim 1 which comprises simultaneously determining the level of free complexing antibody to HIV p17 in the body fluid of the individual.

**4.** Method of claim 3 which comprises simultaneously determining the level of HIV p24, either separately or as the sum of p17 and p24, and also simultaneously determining the level of free complexing antibody to p24 in the fluid.

**5.** Method of claim 1 wherein a heterogeneous sandwich ELISA is used for measuring the level of p17 antigen in the body fluid.

**6.** Method of claim 2 or 3 wherein a p17 binding capacity assay is used for measuring the level of free complexing anti-p17 antibody in the body fluid, the binding capacity assay comprising mixing and incubating a sample of the fluid with a known concentration of exogenous p17 in a buffer to complex any free complexing anti-p17 antibody in the fluid, determining the concentration of unbound exogenous p17 in the fluid by means of a heterogeneous sandwich p17 ELISA , and calculating by difference the concentration of bound p17 in the fluid, which is directly proportional to the concentration of free complexing anti-p17 antibody in the fluid.

**7.** Method of claim 6 wherein the p17 is purified native, synthetic or recombinant p17.

**8.** Method of claim 4 wherein a heterogeneous sandwich ELISA is used for measuring the level of p24 antigen or combined p24 and p17 antigen in the body fluid and a p24 binding capacity assay is used for measuring the level of anti-p24 antibody in the body fluid.

**Patentansprüche**

**1.** Verfahren zum Überwachen und Beurteilen der Entwicklung von AIDS in einer Person, die auf einen Antikörper gegen HIV serumpositiv ist, welches das periodische Bestimmen des HIV-p17-Spiegels in einer Körperflüssigkeit der Person umfaßt.

**2.** Verfahren zum Überwachen und Beurteilen der Entwicklung von AIDS in einer Person, die auf einen Antikörper gegen HIV serumpositiv ist, welches das periodische Bestimmen des Spiegels des freien, komplexierenden Anti-p17-Antikörpers in einer Körperflüssigkeit der Person umfaßt.

**3.** Verfahren des Anspruchs 1, welches das gleichzeitige Bestimmen des Spiegels des freien, komplexierenden Antikörpers auf HIV-p17 in der Körperflussigkeit der Person umfaßt.

**4.** Verfahren des Anspruchs 3, welches das gleichzeitige Bestimmen des HIV-p24-Spiegels entweder getrennt oder als Summe von p17 und p24 und auch das gleichzeitige Bestimmen des Spiegels des freien, komplexierenden Antikörpers auf p24 in der Flüssigkeit umfaßt.

**5.** Verfahren des Anspruchs 1, bei welchem ein heterogener Sandwich-ELISA zum Messen des Spiegels des p17-Antigens in der Körperflüssigkeit verwendet wird.

**6.** Verfahren des Anspruchs 2 oder 3, bei welchem ein Test der p17-Bindungsfähigkeit zum Messen des Spiegels des freien, komplexierenden Anti-p17-Antikörpers in der Körperflüssigkeit benützt wird, wobei der Test der Bindungsfähigkeit das Mischen und Inkubieren einer Probe der Flüssigkeit mit einer bekannten Konzentration an exogenem p17 in einem Puffer unter Komplexieren jeglichen freien, komplexierenden Anti-p17-Antikörpers in der Flüssigkeit, Bestimmen der Konzentration von ungebundenem, exogenem p17 in der Flüssigkeit mittels eines heterogenen Sandwich-p17-ELISA und Berechnen der Konzentration des gebundenen p17 in der Flüssigkeit, welche der Konzentration des freien, komplexierenden Anti-p17-Antikörpers in der Flüssigkeit direkt proportional ist, durch Differenzbildung umfaßt.

**7.** Verfahren des Anspruchs 6, bei welchem das p17 gereinigtes, natives, synthetisches oder rekombinantes p17 ist.

**8.** Verfahren des Anspruchs 4, bei welchem ein heterogener Sandwich-ELISA zum Messen des Spiegels des p24-Antigens oder des kombinierten p24- und p17-Antigens in der Körperflüssigkeit verwendet wird und ein Test der p24-Bindungsfähigkeit zum Messen des Spiegels des Anti-p24-Antikörpers in der Körperflüssigkeit verwendet wird.

**Revendications**

**1.** Procédé pour contrôler et évaluer l'évolution du SIDA chez un individu qui est séropositif pour un anticorps dirigé contre le HIV, qui comprend la détermination par intermittence du taux de p17 de HIV dans un fluide corporel de l'individu.

**2.** Procédé pour contrôler et évaluer l'évolution du SIDA chez un individu qui est séropositif pour un anticorps dirigé contre le HIV, qui comprend la détermination par intermittence du taux d'anticorps anti-p17 complexant libre dans un fluide corporel de l'individu.

**3.** Procédé selon la revendication 1, qui comprend la détermination simultanée du taux d'anticorps complexants libres dirigés contre le p17 du HIV dans le fluide corporel de l'individu.

**4.** Procédé selon la revendication 3, qui comprend la détermination simultanée du taux de p24 du HIV, soit séparément, soit en tant que somme de p17 et p24, et aussi la détermination simultanée du taux d'anticorps complexant libre dirigé contre le p24 dans le fluide.

**5.** Procédé selon la revendication 1, dans lequel un test ELISA hétérogène en sandwich est utilisé pour la mesure du taux d'antigène p17 dans le fluide corporel.

**6.** Procédé selon les revendications 2 ou 3, dans lequel un test de la capacité de liaison de p17 est utilisé pour mesurer le taux d'anticorps anti-p17 complexant libre dans le fluide corporel, le test de la capacité de liaison comprenant le mélange et l'incubation d'un échantillon du fluide avec une concentration connue de p17 exogène dans un tampon pour former un complexe avec tout anticorps anti-p17 complexant libre dans le fluide, la détermination de la concentration de p17 exogène non lié dans le fluide par un test ELISA p17 hétérogène en sandwich du p17 et le calcul par différence de la concentration du p17 lié dans le fluide, qui est directement proportionnelle à l'anticorps anti-p17 complexant libre présent dans le fluide.

**7.** Procédé selon la revendication 6, dans lequel le p17 est un p17 recombinant, de synthèse ou naturel purifié.

**8.** Procédé selon la revendication 4, dans lequel un test ELISA hétérogène en sandwich est utilisé pour la mesure du taux d'antigène p24 ou des antigènes combinés p24 et p17 dans le fluide corporel, et un test de la capacité de liaison de p24 est utilisé pour la mesure du taux d'anticorps anti-p24 dans le fluide corporel.

Figure 1

## p24 antigen standard curve

Figure 2a

## HIV p17 Specific Elisa

Figure 2b

p24 and p17 antigen detection

OD 490 nm

antigen [pg/ml]

p24
P17

Figure 2c

## PATIENT #212 CORE ANTIGENS

## PATIENT #212 CORE BC's

Figure 3

PATIENT #18 CORE ANTIGENS

PATIENT #18 CORE BC's

Figure 4

Figure 5